# EUROPEAN PATENT APPLICATION

(11) **EP 4 295 794 A1**
(43) Date of publication of application: **27.12.2023**
(21) Application number: 23181209.0
(22) Date of filing: 23.06.2023
(51) Int. Cl.: A61B 18/00, A61B 18/14

(54) **PULSED ELECTRIC FIELD DELIVERY DEVICE**

(30) Priority: 24.06.2022 US 202263355169 P; 21.06.2023 US 202318338762
(71) Applicant: Medtronic, Inc., Minneapolis, MN 55432 (US)
(72) Inventor: Kelly, Brian J., Minneapolis 55432 (US); Howard, Brian T., Minneapolis 55432 (US); Laske, Timothy G., Minneapolis 55432 (US); Kenny, Gavin J., Minneapolis 55432 (US); Malewicz, Andrzej M., Minneapolis 55432 (US); Stewart, Mark T., Minneapolis 55432 (US)
(74) Representative: Zimmermann & Partner Patentanwälte mbB

(57) **Abstract**

A medical device includes an elongate body, an expandable treatment element, a plurality of flexible shafts, and a plurality of electrodes. The elongate body has a proximal portion and a distal portion opposite the proximal portion. The expandable treatment element is coupled to the elongate body to receive a fluid and, in some examples, is anchored to the plurality of flexible shafts with a plurality of retention elements. In some examples, each flexible shaft of the plurality of flexible shafts has a braided configuration. Each electrode of the plurality of electrodes is attached and electrically coupled to a respective flexible shaft of the plurality of flexible shafts.

## Description

### CROSS-REFERENCE TO RELATED APPLICATION

This application claims the benefit of U.S. Provisional Patent Application No. 63/355,169, filed 24-JUNE-2022, and entitled "PEF DELIVERY DEVICE."

### TECHNICAL FIELD

The present disclosure relates to methods, systems, and devices for enhancing the efficiency and efficacy of ablation energy delivery and improving patient safety.

### BACKGROUND

Tissue ablation is used in numerous medical procedures to treat a patient. Ablation can be performed to remove undesired tissue such as cancer cells. Ablation procedures may also involve killing the tissue without removal, such as to stop electrical propagation through the tissue in patients with an arrhythmia. The ablation is often performed by passing energy, such as electrical energy, through one or more electrodes causing the tissue in contact with the electrodes to heat up to an ablative temperature but may also be performed by freezing the tissue with the use of a cryoablation catheter. Pulsed field ablation (PFA) is a more advanced form of ablation that uses pulsed electric fields delivered to the diseased tissue to cause cell death by the process of irreversible electroporation.

### SUMMARY

Medical procedures, such as cardiac ablation using one or more energy modalities, are frequently used to treat such conditions. For example, some devices include electrodes configured to deliver electrical energy to a target treatment area. However, some of those devices do not provide adequate dielectric protection or separation between circuits, which may lead to electrical shortening. Further, the electrodes in some devices are either disposed directly on a balloon element or disposed on structurally weak and/or flexible structures that may become displaced during tissue contact from their intended radial spacing, thereby adversely affecting certain important characteristics of the generated electric fields.

The techniques of this disclosure generally relate to methods, systems, and devices for enhancing the efficiency and efficacy of ablation energy delivery and improving patient safety.

One example provides a medical treatment apparatus for delivering pulsed electric field (PEF) energy to a target tissue. The medical treatment apparatus includes an elongate body having one or more lumens for mechanical, electrical, and fluid communication between a proximal portion and a distal portion. The distal portion includes a plurality of flexible shafts arranged around an actuation element and coupled between the elongate body and a distal tip section of the actuation element such that longitudinal movement of the actuation element with respect to the elongate body flexes the flexible shafts. The medical treatment apparatus also includes an expandable element attached to the distal tip section and positioned within a space delimited by the flexible shafts. The expandable element is connected via the one or more lumens to receive a fluid. The medical treatment apparatus also includes a plurality of electrodes arranged along the flexible shafts and electrically connected via the one or more lumens to receive PEF energy for delivery to the target tissue.

In one aspect, a medical device includes an elongate body, an expandable treatment element, a plurality of flexible shafts, and a plurality of electrodes. The elongate body has a proximal portion and a distal portion opposite the proximal portion. The expandable treatment element is coupled to the elongate body. The plurality of flexible shafts is coupled to the expandable treatment element. The plurality of flexible shafts each have a braided configuration for structural reinforcement. The plurality of electrodes is coupled to each shaft of the plurality of flexible shafts.

In another aspect, the plurality of flexible shafts are each spaced apart from an adjacent shaft at a distance sufficient to prevent electrical shorting.

In another aspect, the medical device further includes an actuation element slidably disposed within the elongate body.

In another aspect, retraction of the actuation element causes the expandable treatment element to transition from a first configuration to a second configuration.

In another aspect, a distal portion of the actuation element defines a distal tip.

In another aspect, the distal tip extends distally beyond a distal face of the expandable element when in the first configuration. The distal top does not extend distally beyond the distal face of the expandable treatment element when in the second configuration.

In another aspect, each of the plurality of electrodes is configured for delivering pulsed electric field (PEF) energy to an area of target tissue.

In another aspect, at least a portion of each of the plurality of electrodes is coated with an insulative material.

In another aspect, the expandable treatment element is a balloon.

In yet another embodiment, a medical system includes an energy generator and a medical device having an elongate body, an expandable treatment element, a plurality of flexible shafts, and a plurality of electrodes. The elongate body has a proximal portion and a distal portion opposite the proximal portion. The expandable treatment element is coupled to the distal portion of the elongate body. The plurality of flexible shafts is coupled to the expandable treatment element, and each have a braided configuration for structural reinforcement. The plurality of electrodes is coupled to each shaft of the plurality of flexible shafts. The energy generator is in electrical communication with the medical device.

In another aspect, the energy generator includes processing circuitry. The processing circuitry is configured to initiate a delivery of pulsed electric field (PEF) energy from the energy generator to the plurality of electrodes.

In another aspect, the plurality of flexible shafts is each spaced apart from one another and the adjacent shaft at a distance sufficient to prevent electrical shorting.

In another aspect, the medical device further includes an actuation element slidably disposed within and at least partially extending from the distal portion of the elongate body. The actuation element is partially disposed within the expandable treatment element.

In another aspect, retraction of the actuation element causes the expandable treatment element to transition from a first configuration to a second configuration.

In another aspect, a distal portion of the actuation element defines a distal tip.

In another aspect, the distal tip extends distally beyond a distal face of the expandable element when in the first configuration. The distal tip does not extend distally beyond the distal face of the expandable treatment element when in the second configuration.

In another aspect, each of the plurality of electrodes is configured for the delivery of pulsed electric field (PEF) energy.

In another aspect, at least a portion of each of the plurality of electrodes is coated with an insulative material.

In another aspect, the expandable treatment element is a balloon.

In one aspect, a catheter electrode distribution system (CEDS) is configured to initiate a delivery of pulsed electric field (PEF) energy from the energy generator to the plurality of electrodes in different directional vectors.

In one aspect, a non-tissue contacting recording electrode is configured to be in electrical communication with each of the plurality of electrodes to generate a monophasic action potential recording.

In yet another embodiment, a medical system includes an energy generator and a medical device having an elongate body, an actuation element, an expandable treatment element, a plurality of flexible shafts, and a plurality of electrodes. The elongate body has a proximal portion and a distal portion opposite the proximal portion. The actuation element is slidably disposed within the elongate body and defines a distal tip. The expandable treatment element is coupled to the elongate body. Retraction of the actuation element causes the expandable treatment element to transition from a first configuration to a second configuration. The plurality of flexible shafts is coupled to the expandable treatment element, and each have a braided configuration for structural reinforcement. Each shaft is spaced apart from an adjacent shaft at a distance sufficient to prevent electrical shorting. The plurality of electrodes is coupled to each shaft of the plurality of flexible shafts and are configured to deliver pulsed electric field (PEF) energy to an area of target tissue. At least a portion of each electrode is coated with an insulative material. The electrical generator is in electrical communication with the medical device, the energy generator includes processing circuitry configured to initiate a delivery of PEF energy from the energy generator to the plurality of electrodes.

In yet another embodiment is a method of ablating tissue within a patient. The method includes positioning a medical device proximate to an area of target tissue. The medical device includes an elongate body and an expandable treatment element coupled to the elongate body. The expandable treatment element having a plurality of flexible shafts coupled to an exterior surface of the expandable treatment element. The plurality of flexible shafts each having a braided configuration for structural reinforcement. The method further includes inflating the expandable treatment element during an inflation phase and transitioning the expandable treatment element from a first extended configuration to a second retracted configuration.

In one aspect, the medical device further includes a plurality of electrodes coupled to each shaft of the plurality of flexible shafts.

In one aspect, transitioning the expandable treatment element from the first extended configuration to the second retracted configuration includes retracting an actuation element at least partially disposed within the elongate body.

In one aspect, a distal portion of the actuation element defines a distal tip.

In one aspect, the distal tip extends distally beyond a distal face of the expandable treatment element when in the first configuration, and the distal tip does not extend distally beyond the distal face of the expandable element when in the second configuration.

In one aspect, the method further includes advancing the medical device within the patient such that the expandable treatment element is in contact with the area of target tissue when in the second retracted configuration.

In one aspect, the method further includes delivering pulsed electric field (PEF) energy from an energy generator to each of the plurality of electrodes.

In one aspect, at least a portion of each of the plurality of electrodes is coated with an insulative material.

In yet another embodiment is a medical system including an elongate body, an expandable treatment element coupled to the elongate body, and a plurality of flexible shafts each having a braided configuration for structural reinforcement.

In one aspect, the plurality of flexible shafts or splines together define a volume, the expandable treatment element encompassing less than the full volume of the plurality of flexible shafts.

In one aspect, the plurality of flexible shafts each have a proximal end and a distal end opposite the proximal end. The expandable treatment element is coupled only to the distal end of each flexible shaft.

In one aspect, the distal end of each flexible shaft is proximate to the distal tip.

In one aspect, a plurality of shaft retention elements is coupled to the expandable treatment element and are disposed around an outer surface of each of the plurality of flexible shafts.

In one aspect, a plurality of electrodes is coupled to each shaft of the plurality of splines and are configured to deliver pulsed electric field (PEF) energy to an area of target tissue.

In one aspect, at least one shaft or spline retention element is positioned between a first electrode and a second electrode of the plurality of electrodes.

In one aspect, at least a portion of each of the plurality of electrodes is coated with an insulative material.

In one aspect, the expandable treatment element defines at least one pore.

In one aspect, the expandable treatment element is flexible when inflated.

In one aspect, the medical device further includes a handle.

In one aspect, the plurality of flexible shafts each include a lumen and a super-elastic stiffening wire disposed within each lumen, the stiffening wire being movable longitudinally within each lumen and coupled to the handle of the medical device.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF THE DRAWINGS

A more complete understanding of the present invention, and the attendant advantages and features thereof, will be more readily understood by reference to the following detailed description when considered in conjunction with the accompanying drawings wherein:
FIGS. 1A-1C are schematic diagrams illustrating a medical system according to various examples.
FIG. 2 shows a side view of a distal portion of the medical device used in the medical system of FIGS. 1A-1C in a first extended configuration according to some examples.
FIG. 3 shows a side view of the distal portion the medical device used in the medical system of FIGS. 1A-1C in a second retracted configuration according to some examples.
FIG. 4 shows a front view of the distal portion of the medical device of FIGS. 1-3 wherein a plurality of braided shafts contour around an outer surface of an expandable element towards a distal tip in a first configuration according to some examples.
FIG. 5 shows a front view of the distal portion of the medical device of FIGS. 1-3 wherein the plurality of braided shafts contour around the outer surface of the expandable element towards the distal tip in a second configuration according to some examples.
FIG. 6 shows a plurality of electrodes disposed along the plurality of shafts illustrated in FIGS. 1-5 and coated with an electrically insulative material according to some examples.
FIG. 7 shows a side view of a distal portion of a medical device in a first extended configuration, the medical device including a plurality of shaft retention elements being disposed around a plurality of shafts according to some examples.
FIG. 8 shows a side view of the distal portion of the medical device of FIG. 7 in a second retracted configuration according to some examples.
FIG. 9 shows a front view of the distal portion of the medical device of FIGS. 7-8, wherein the plurality of shafts contour around an outer surface of the expandable element towards a distal tip in a first configuration according to some examples.
FIG. 10 shows a side view of the medical device of FIGS. 7-9 in a third retracted configuration according to some examples.
FIG. 11 shows a flow chart of an example method performed in accordance with various embodiments.
FIGS. 12A-12B illustrate a braided shaft used in the medical devices of FIGS. 2-10 according to some examples.

### DETAILED DESCRIPTION

The devices, systems, and methods disclosed herein provide for methods, systems, and devices for enhancing the efficiency and efficacy of ablation energy delivery and for improving patient safety. Further, described herein are device and system configurations and energy delivery patterns that facilitate irreversible electroporation of target tissue cells by delivering electrical field energy to the target tissue. The devices and systems described herein enhance patient safety and increase ablation efficiency by allowing for increased concentration of electrical energy from the treatment device when in contact with target tissue.

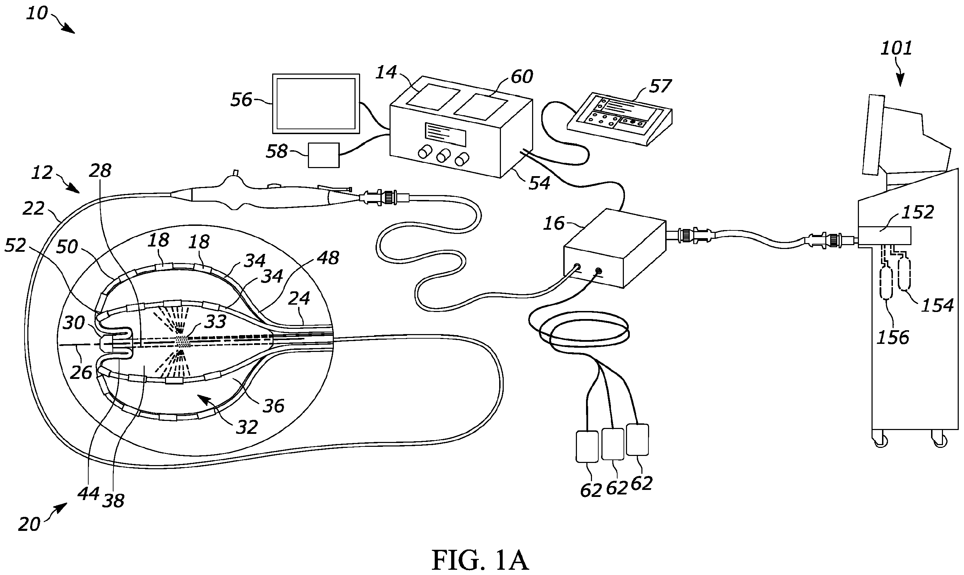

FIGS. 1A-1C are schematic diagrams illustrating a medical system 10 according to several examples. More specifically, three examples of the medical system 10 shown in FIGS. 1A-1C have different respective fluid delivery subsystems. Hence, the following description of the medical system 10 first focuses on the system parts that are common to all three shown examples. Thereafter, the different respective fluid delivery subsystems are described with specific reference to individual ones of FIGS. 1A-1C.

Referring now to collectively to FIGS. 1A-1C, the system 10 may generally include a treatment or medical device 12, such as a catheter, that may be coupled directly to an energy supply, such as an electroporation energy generator 14 including an energy control, delivering, and monitoring system, or indirectly through a device electrode and fluid distribution system 16 (which may also be referred to herein as a catheter electrode distribution system or CEDS). Further, the medical device 12 may include one or more diagnostic or treatment regions for the energetic, therapeutic, and/or investigatory interaction between the medical device 12 and a treatment site. As a non-limiting example, the treatment region(s) may include a plurality of electrodes 18 configured to deliver electroporation energy to a tissue area in proximity to the electrodes 18. Although the system 10 is discussed herein as being used for electroporation through pulsed field ablation with the option to supplement this with cryoablation, it will be understood that the medical device 12, generator 14, and/or other system components may additionally or alternatively be configured for use with radiofrequency (RF) ablation and the like.

The medical device 12 may serve both as a treatment device and a mapping device. The medical device 12 may include an elongate body 20 passable through a patient's vasculature and/or proximate to a tissue region for diagnosis and/or treatment. For example, the medical device 12 may be a catheter that is deliverable to the tissue region via a sheath or intravascular introducer (not shown). The elongate body 20 may define a proximal portion 22, a distal portion 24, and a longitudinal axis 26, and may further include one or more lumens disposed within the elongate body 20 thereby providing mechanical, electrical, and/or fluid communication between the elongate body proximal portion 22 and the elongate distal portion 24.

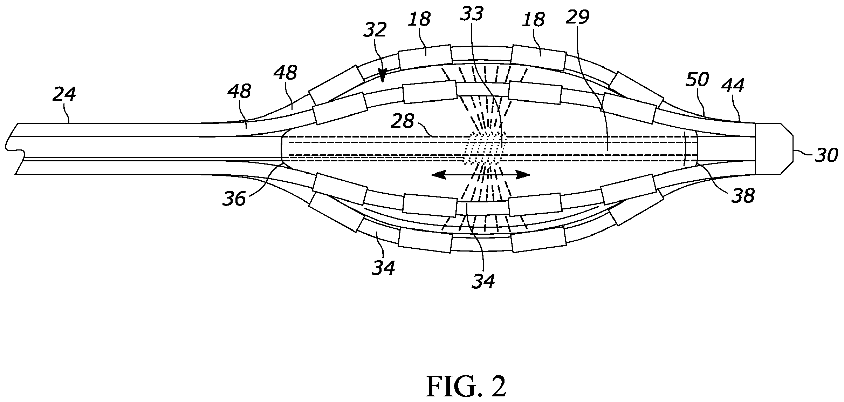

The medical device 12 may include a rigid or semi-rigid shaft or actuation element 28 at least partially disposed within a portion of the elongate body 20. The actuation element 28 may extend or otherwise protrude from a distal end of the elongate body 20 and may be movable with respect to the elongate body 20 in longitudinal and rotational directions. That is, the actuation element 28 may be slidably and/or rotatably moveable with respect to the elongate body 20. The actuation element 28 may further define a lumen (e.g., see element 29 in FIG. 2) therein for the introduction and passage of a guide wire (not shown). The actuation element 28 may comprise a plurality of sections, each section having a different respective diameter, with the actuation element 28 terminating in or otherwise including an area having a larger diameter than the rest of the actuation element 28, which may be referred to as a distal tip 30 (which is included at the distal end of the distal portion 24). The distal tip 30 may be defined at a distal end of the actuation element 28 and may define an opening and passage therethrough that is in communication with the shaft lumen 29. As discussed in greater detail below, it will be understood that the actuation element 28 may have a single continuous diameter with an expandable element 32 being attached to the actuation element 28 proximate the distal end of the actuation element 28.

The medical device 12 may further include one or more expandable elements at, coupled or affixed to, or otherwise on the elongate body distal portion 24 for energetic, therapeutic, diagnostic and/or investigatory interaction between the medical device 12 and a treatment site or region. As a non-limiting example, the expandable element 32 may be a highly conformable and expandable balloon, such as the balloon illustrated in FIGS. 2-3. A fluid delivery conduit 33 in fluid communication with the fluid delivery subsystem and is provided to release a fluid, such as refrigerant, gas, or saline, from one or more openings, apertures, or ports defined in the conduit 33 within the expandable element 32 in response to console commands and/or other electrical or mechanical control input. As shown in FIGS. 2-3, the fluid delivery conduit 33 extends within the expandable element 32 parallel to the actuation element 28 and is coupled to the actuation element 28. In some examples, the expandable element 32 can be filled with a fluid containing one or more therapeutic agents (such as genes, RNA, drugs, etc.) deliverable to the surrounding tissue, for example, through pores in an outer wall (e.g., a skin or film) of the expandable element 32.

In some configurations, the fluid delivery conduit 33 may be disposed circumferentially, spirally, and/or helically around the outer surface of the actuation element 28 such that the one or more ports face away from the actuation element 28 and towards the inner surface of the expandable element 32. However, in other configurations not shown, the actuation element 28 may instead define the one or more ports located within an interior or inner chamber of the expandable element 32 such that fluid may be delivered to inflate the expandable element 32 in the absence of the fluid delivery conduit 33. Additionally, in some configurations, the fluid delivery conduit 33 may be a lumen or tube that extends within the inner chamber of the expandable element 32 but is not mechanically coupled to the actuation element 28.

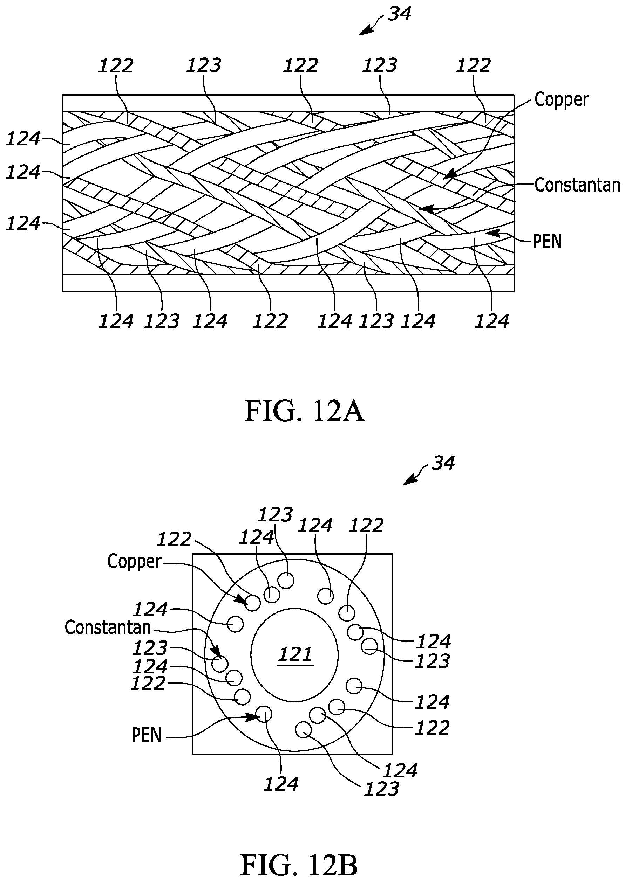

The medical device 12 may also include a plurality of flexible shafts or splines 34 disposed on and/or contouring around an outer surface of the expandable element 32 between a proximal end 36 and a distal end 38 of the expandable element 32 or the distal tip 30. In some examples, the splines 34 are braided for structure or provided with a central super-elastic structural wire surrounded by a polymeric cover which also provides a supporting surface for the electrodes (e.g., see FIGS. 12A-12B). The braiding, however, allows for a smaller diameter super-elastic member to be incorporated, thus beneficially decreasing the overall size of each spline. This feature allows some of the wires to be included within the lumen. Also contained within the polymeric lumen are the electrical wires that connect to each electrode.

In one illustrated embodiment, the expandable element 32 includes a proximal neck defined at the proximal end 36 and a distal neck defined at the distal end 38. The shafts 34 are positioned around the outer surface of the expandable element 32 such that a distal end 44 of each shaft 34 is proximate to, coupled to, or in contact with, the distal neck. In one embodiment, the shafts 34 may be uniformly spaced around the outer surface of the expandable element 32 or they may be spaced in any configuration necessary to achieve a desired ablation pattern. The shafts 34 may be formed by braided electrical and/or mechanical wiring (including tracings) that are coupled to the outer surface of the expandable element 32.

In some examples, the construction of the multiple splines includes a central super-elastic member combined with either an intertwined or braided jacket. In some other examples, the jacket is spiral wrapped with structural wires or the spiral wrapped wires within the jacket serve as electrical conductors, each being an individually insulated wire contained (embedded) in a spiral wrap within the outer jacket of each spline structure. In another configuration, the wires run in a linear fashion through the splined structure jacket wall. Variations on running the wires straight through (within) the jacket walls or slightly spiraled are also used in some examples. Also, it may be important to include an option that in either braided or spiral wrapped configurations of wires within the jacket walls, some of the "strands" can be non-conductive polymeric fibers, such as polyethylene naphthalate (polyethylene 2,6-naphthalate) or PEN) or Aramid fibers. In braided configurations, non-conductive fibers can be provided in one of the directions of the wrap, such that none of the overlapping points of the reinforcing braid are metal over metal. That configuration can improve electrical reliability if some of the braid "strands" are used as electrical conductors. In such examples, each overlap point within the jacket braid has a metal wire in contact with non-conductive fibers (typically polymeric).

The braided configuration of the shafts 34 reduce the profile of the shafts 34 and allow more wiring to be included in each shaft 34. In some devices and systems, splines generally are made of a super-elastic internal structural shaping wire. However, the wiring of each braided shaft 34 is used as a mechanical reinforcement for each shaft 34, thereby allowing for the internal super-elastic structural wire to be eliminated altogether or reduced in size, thus further reducing the profile of the medical device 12. Additionally, because the wiring is contained within the braided shafts 34, they do not require space within a lumen of the elongate body 20 to pass through, thereby allowing for a reduction in cross-sectional size of each shaft 34. Further, the shafts 34 may allow for smaller electrode size and enhancement of mapping signal recording.

Referring to FIG. 1A, the CEDS 16 is connected to a cryo-console 101 including a refrigerant source 154 and a vacuum pump 156. A plumbing and valve box 152 is configured to controllably connect and disconnect the refrigerant source (e.g., a pressurized bottle) 154 and the vacuum pump 156 to the corresponding lumens running to the CEDS 16 and further to the medical device 12 to produce and maintain a desired pressure in the expandable element 32. In some examples, the plumbing and valve box 152 is operated to also achieve a desired (e.g., relatively low) temperature in the expandable element 32. In some examples, the plumbing and valve box 152 may contain a relatively large number of components, such as flow and/or pressure monitors, control valves, and one or more additional pumps. In operations, the vacuum pump 156 is used to pull a continuous flow of the refrigerant back from the expandable element 32.

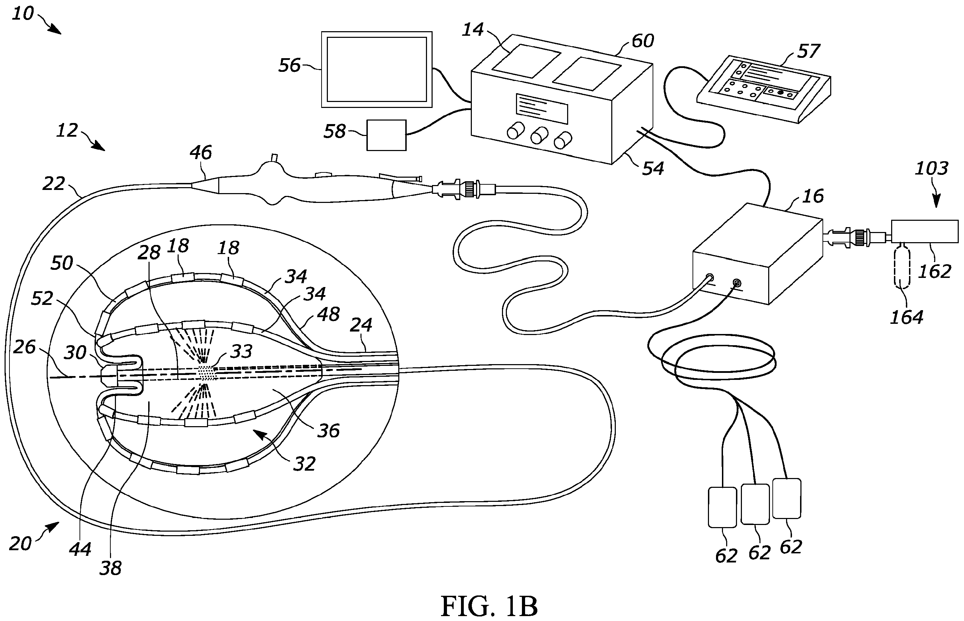

Referring to FIG. 1B, the CEDS 16 is connected to a fluid or gas supply source 103. The supply source 103 includes a pressurized bottle 164 and a fluid or gas supply controller 162. In operation, the controller 162 regulates the flow of fluid or gas from the pressurized bottle 164 to the expandable element 32, thereby controlling the pressure therein. In various examples, the fluid or gas supply controller 162 includes one or more of the following: means for retracting the fluid/gas from the expandable element 32, a pump, an automated syringe, a flow monitor, and one or more control valves.

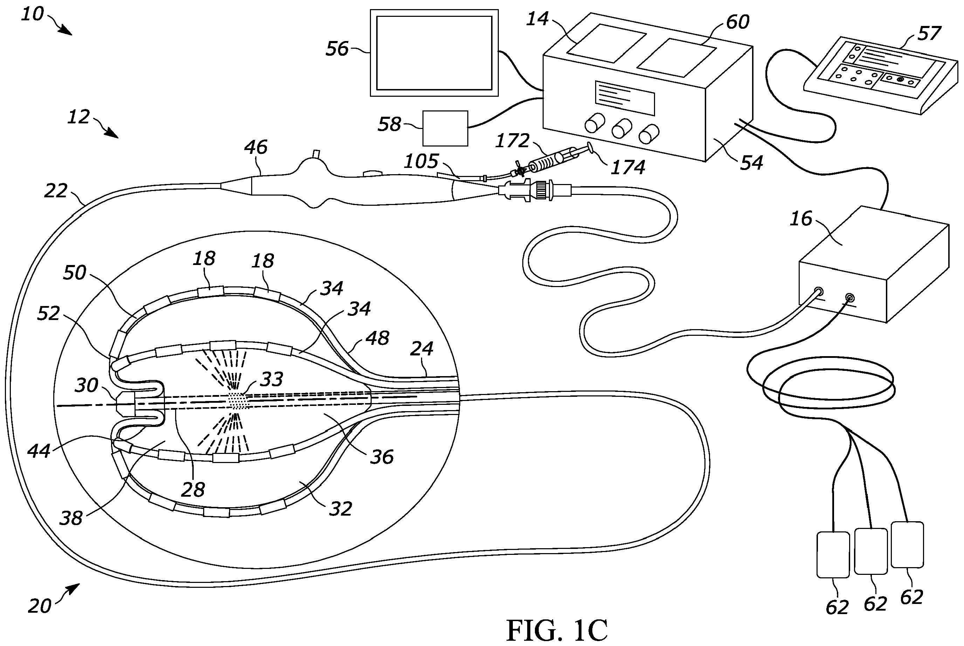

Referring to FIG. 1C, a fluid injection port 105 on a handle 46 of the medical device 12 is connected to a syringe 172 having a plunger 174. The barrel of the syringe 172 is filled with a liquid or gas, which can be transferred to or withdrawn from the expandable element 32, via the fluid delivery conduit 33, by appropriately moving the plunger 174. In some examples, instead of being connected to the fluid injection port 105 on the handle 46, the syringe 172 is connected to the CEDS 16, e.g., similar to the supply source 103 of FIG. 1B. In cases of liquid (e.g., saline) injections, the terminal port of the fluid delivery conduit 33 in the expandable element 32 preferably has a simpler configuration than the helical configuration shown in FIG. 1C. For example, a round hole in the liquid delivery shaft is sufficient for injecting and retracting the liquid to/from the expandable element 32 with the syringe 172.

Continuing to refer to FIGS. 1A-1C and 2-3, the medical device 12 may include at least one electrode 18 coupled to, mounted to, adhered to, affixed to, or otherwise integrated with the material of each shaft of the plurality of shafts 34 around the outer surface of the expandable element 32. Each electrode 18 may be in electrical communication with the generator 14 and/or any other electrodes 18 via the electrical tracings included in the braided shafts 34. The electrodes 18 may be composed of any suitable electrically conductive material(s), such as metals and/or metal alloys. In one embodiment, each shaft 34 may include a plurality of electrodes 18 spaced along the length of each shaft 34 around the outer surface of the expandable element 32 such that the electrodes 18 may be in contact with an area of target tissue. The electrodes 18 may be uniformly equally spaced about the length of each shaft 34 or they may be positioned along the shafts 34 in any suitable configuration to achieve a desired ablation effect. Because the braided configuration of each shaft 34 allows for a reduced profile, each electrode 18 may be positioned closer together along each shaft 34. The braided configuration also allows the shafts 34 to be more flexible and readily contort to match the outer surface of the expandable element 32 during navigation phases and/or treatment phases. Further, it is to be understood that at least some of the plurality of electrodes 18 may also be located on a portion of each shaft 34 that is not in direct contact with tissue when the medical device 12 is in use.

As shown in FIG. 2, the distal tip 30 extends distally beyond the distal end 38 of the expandable element 30. The distal tip 30 may also include one or more electrodes 18. All electrodes 18 of the system may be in electrical communication with the generator 14. Thus, energy may be delivered between one or more of the electrodes 18 on the shafts 34 and/or the distal tip 30 to create different ablation patterns, such as ablation patterns that are linear or extended in a proximal-to-distal direction instead of or in addition to circumferential ablation patterns. In one example configuration, the CEDS 16 may initiate the delivery of energy from the generator 14 between selected electrodes 18 in different directional vectors, including between electrodes 18 on the same shaft 34, electrodes 18 on adjacent shafts 34, and electrodes 18 on the shafts 34 and the distal tip 30.

In some examples, the handle 46 of the medical device 12 includes circuitry for identification and/or use in controlling of the medical device 12 or another component of the system. Additionally, the handle 46 also includes connectors that are mateable to the generator 14 and/or the CEDS 16 to establish communication between the medical device 12 and the generator 14. The handle 46 may also include one or more actuation or control features that allow a user to extend, retract, deploy, control, deflect, steer, or otherwise manipulate a distal portion of the medical device 12 from the proximal portion of the medical device 12.

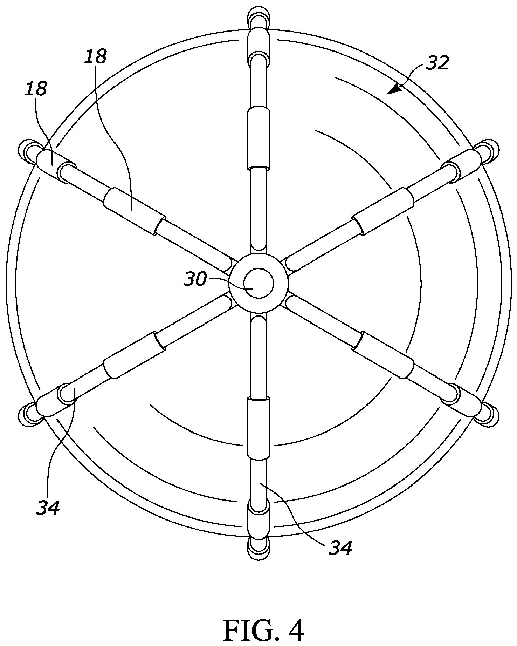

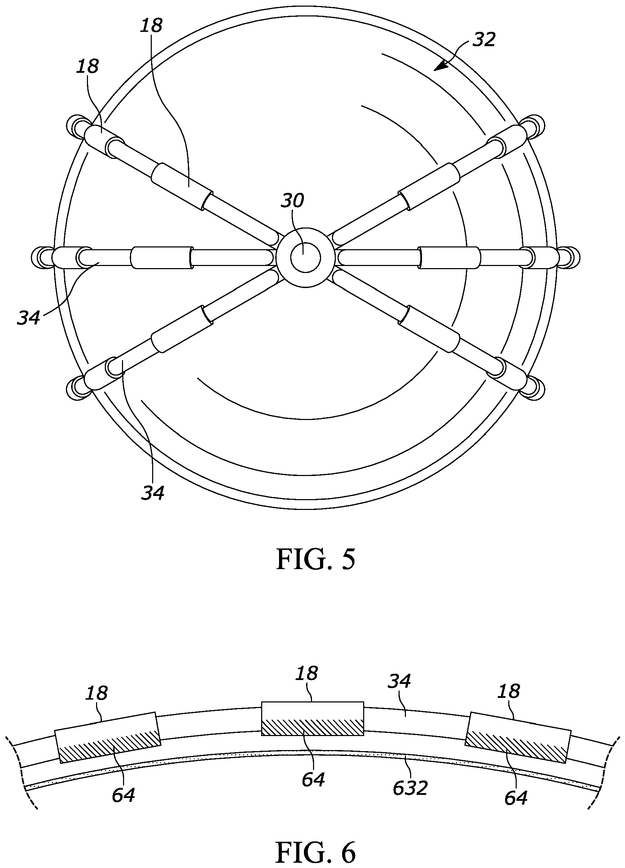

In one example embodiment, the medical device 12 includes six shafts 34 (as shown in FIGS. 4 and 5), each shaft 34 having a proximal portion 48 and a distal portion 50. The distal portion 50 of each shaft 34 includes electrodes 18. In one non-limiting example, each of the six shafts 34 each includes four electrodes 18 such that the device 12 includes twenty-four electrodes 18 disposed amongst six shafts 34. The proximal portion 48 of each shaft, and the portions of the distal portion 50 of each shaft 34 located between electrodes 18, optionally may be insulated (for example, may include an insulative coating). In one non-limiting example, as shown in FIG. 4, the six shafts 34 are uniformly spaced apart around the expandable element 32. In another non-limiting example, as shown in FIG. 5, the six shafts 34 are grouped around the outer surface of the expandable element 32 in sets. For example, the device 12 may include two sets of shafts 34. The first set includes three shafts 34, and the second set also includes three shafts 34. The first and second sets may be separated about the expandable element 32 such that the first set of shafts 34 is opposite the second set of shafts 34. It is to be understood that the plurality of shafts 34 is not limited to six shafts. Rather, the plurality of shafts may include any suitable number of shafts to achieve a desired treatment effect. For example, the plurality of shafts 34 may include 2, 4, 5, 6, 7, 8, etc., shafts. Additionally, the shafts 34 may be grouped in any suitable manner such that the first set of shafts may have a greater, lesser, or equal number of shafts as the second set of shafts.

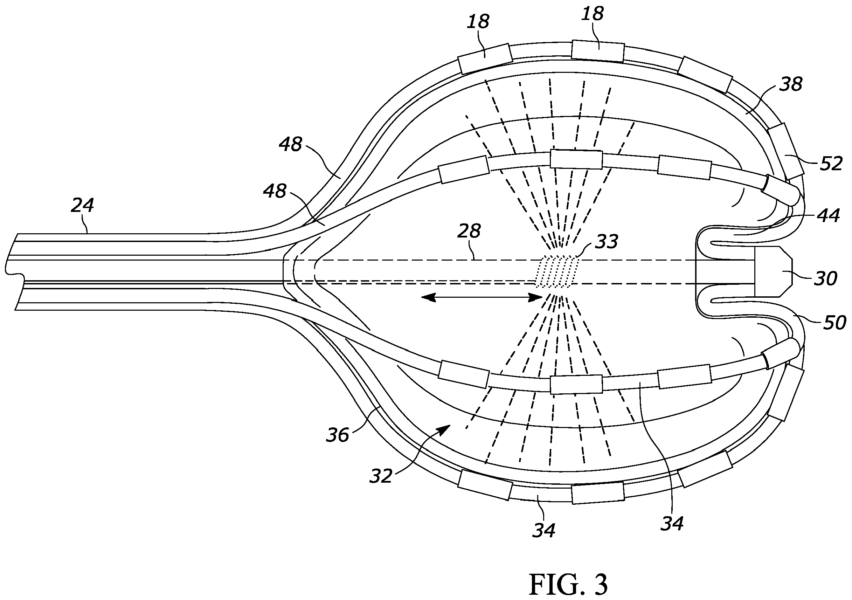

Once again referring to FIGS. 2 and 3, the distal portion 50 of each shaft 34 such as the distal end 44 of each shaft 34, may be adhered to, affixed to, or otherwise coupled to the distal neck of the expandable element 32 or distal tip 30. Longitudinal movement of the actuation element 28 within the elongate body 20 may change the size, shape, and/or configuration of the shaft(s) 34. For example, advancement of the actuation element 28 within the elongate body 20 may extend the shafts 34 and reduce the diameter of the expandable element 32, whereas retraction of the actuation element 28 within the elongate body 20 may retract the shafts 34 and increase the diameter of the expandable element 32. That is, the expandable element 32 is transitionable from a first extended configuration (as shown in FIG. 2) in which the distal tip 30 protrudes distally beyond the expandable element 32 to a second retracted configuration in which each shaft 34 is curvilinear, bowed, or arcuate, and the distal tip 30 is retracted towards the handle 46 such that the expandable element 32 forms a distal face 52 (e.g., as shown in FIG. 3). The distal face 52 may be formed by the distal neck of the expandable element 32 becoming inverted as the actuation element 28 is retracted and the distal neck is drawn towards the elongate body 20. In this second retracted configuration, the expandable element 32 may define the distal face 52. Depending on the manufactured shape and configuration of the expandable element 32, the largest or maximum outer diameter of the expandable element 32 may lie at a point that is proximal to the distal face 52, as shown in FIG. 3. The shape and diameter of the expandable element 32 may also depend on the medical procedure for which it will be used. As shown in FIG. 3, in the retracted position, the distal tip 30 may be partially or fully surrounded by at least a portion of the distal end 38 of the expandable element 32 such that the expandable element 32 may be referred to as being "tip-less". The retraction of the distal tip 30 allows for easier navigation and placement of the device 12 at the desired treatment site within the patient. In this retracted position, the distal ends 38 of each shaft 34 may curve inwards towards the center of the expandable element 32. By using the actuation or control features of the handle 46, the clinician may then return the expandable element 32 to its original extended position (for example, as shown in FIG. 2). The flexibility of the braided wiring allows the shafts 34 to readily transition between the retracted and deployed positions without snapping, breaking, cracking, or otherwise being damaged by the expandable element's 32 contortions and movement. In one non-limiting example, the distal tip 30 is permanently retracted within the expandable element 32 such that the clinician is not required to engage the handle 46 to retract the distal tip 30 prior to treating the target tissue.

In some examples, the energy generator 14 is within or in electrical communication with a control unit 54 that further includes or is in electrical communication with one or more other system components, such as one or more displays 56, user input devices 57, a mapping and/or navigation system 58 (which may also be referred to herein as a recording system 58), the CEDS 16, and so on. In addition to being configured to deliver ablation energy, such as electroporation energy, the plurality of electrodes 18 may also be configured to perform diagnostic functions, such as to collect intracardiac electrograms (EGMs) and/or monophasic action potentials (MAPs) as well as performing selective pacing of intracardiac sites for diagnostic purposes. Recorded signals may be transferred from the CEDS 16 to the control unit 54. Alternatively, in some embodiments, the recorded signals may be transferred directly from the medical device 12 to the control unit 54 (for example, to the energy generator 14).

The plurality of electrodes 18 may also be configured to record impedance measurements from tissue and/or fluids surrounding and/or in contact with the electrodes 18 in order to monitor the proximity to target tissues and quality of contact with, for example, an area of target tissues. The plurality of electrodes 18 may also be configured to perform impedance measurements from tissue before, during, and/or after the delivery of energy to determine or qualify lesion formation in the target tissue. The generally accepted definition of the term impedance is used herein: a complex ratio of sinusoidal voltage to current in an electric circuit or component, except that as used herein, impedance shall apply to any region or space through which some electrical field is applied and current flows. The generator 14 may be configured to sense the impedances between selected pairs of the electrodes 18 and use the impedance measurements to activate a selected electrode 18 or deactivate a selected electrode 18. Electrodes 18 may be activated based upon the impedance measurements during ablation. When targeted tissue is identified with the impedance measurement, energy can be delivered to those electrodes in close proximity or in contact with specified tissue and electrodes 18 which are in contact with blood or tissue that is not desirable may be deactivated based upon a specific impedance measurement. The CEDS 16 may include high speed relays to disconnect/reconnect specific electrodes 18 of the plurality of electrodes 18 from/to the generator 14 during an energy delivery procedure. In a non-limiting example, the relays may automatically disconnect/reconnect electrodes 18 to enable the medical device 12 to record mapping or navigational signals between deliveries of energy pulses.

The system 10 may include one or more sensors to monitor the operating parameters throughout the system, in addition to monitoring, recording or otherwise conveying measurements or conditions within the medical device 12 or the ambient environment at the distal portion of the medical device 12. For example, each electrode 18 may include a temperature sensor, pressure sensor, or other sensor. The sensor(s) may be in communication with the generator 14 and/or the CEDS 16, e.g., for initiating or triggering one or more alerts and/or therapeutic delivery modifications during operation of the medical device 12.

Electroporation is a phenomenon causing cell membranes to become "leaky" (that is, permeable for molecules for which the cell membrane may otherwise be impermeable or semipermeable). Electroporation, which may also be referred to as electro-permeabilization, pulsed field ablation, non-thermal irreversible electroporation, irreversible electroporation, high frequency irreversible electroporation, nanosecond electroporation, or nano-electroporation, involves the application of high-amplitude pulses to cause physiological modification (i.e., permeabilization) of the cells of the tissue to which the energy is applied. These pulses preferably may be short (for example, nanosecond, microsecond, or millisecond pulse width) in order to allow the application of high voltage, high current (for example, 20 or more amps) without long duration(s) of electrical current flow that may cause significant tissue heating and muscle stimulation. The pulsed electric energy may induce the formation of microscopic defects that result in hyper-permeabilization of the cell membrane. Depending on the characteristics of the electrical pulses, an electroporated cell can survive electroporation, referred to as "reversible electroporation," or die, referred to as "irreversible electroporation" (IEP). Reversible electroporation may be used to transfer agents, including genetic material and other large or small molecules including but not limited to therapeutic agents, into targeted cells for various purposes, including the alteration of the action potentials of cardiac myocytes.

As such, the control unit 54 may include processing circuitry 60 that includes software modules containing instructions or algorithms to provide for the automated and/or semi-automated operation and performance of various system 10 functions. For example, the processing circuitry 60 may include a processor and a memory in communication with the processor, and the memory may include instructions that, when executed by the processor, configure the processor to perform sequences, calculations, or procedures described herein and/or required for a given medical procedure. In one embodiment, the processing circuitry 60 is a component of the generator 14 within the control unit 54. The processing circuitry 60 may be further configured to deliver electroporation energy or another type of energy to the electrodes 18 and determine whether an alert condition is present. The alert condition may be based at least in part on signals received from the electrode(s) 18 (for example, impedance measurements recorded by the electrode(s) 18) and/or one or more other system sensors. In one embodiment, the generator 14 may be configured to cease the delivery of electroporation energy to one or more electrodes 18 and/or prevent the delivery of electroporation energy to one or more electrodes 18 when the processing circuitry determines the alert condition is present.

The system 10 may further include a plurality of surface electrodes 62 in communication with the generator 14 directly or indirectly through the CEDS 16. The plurality of surface electrodes 62 may be part of the mapping and navigation or recording system 58 that allows for the localization of the electrodes within three-dimensional space within the patient's body through the transmission and receipt of positioning and navigation signals to and from the generator 14. Also, when the surface electrodes 62 are applied to the skin of a patient, they may be used, for example, to monitor the patient's cardiac activity to determine pulse train delivery timing at the desired portion of the cardiac cycle (that is, to record and transmit electrical activity measurements to the generator 14 and/or for navigation and location of the device 12 within the patient). The surface electrodes 62 may be in communication with the generator 14 for determining the timing during a cardiac cycle at which to initiate or trigger one or more alerts or therapeutic deliveries during operation of the medical device 12. In addition to monitoring, recording, or otherwise conveying measurements or conditions within the medical device 12 or the ambient environment at the distal portion 24 of the medical device 12 (for example, electrocardiogram or ECG signals and/or monophasic action potentials or MAPs), the surface electrodes 62 may be used to perform measurements of one or more of temperature, electrode tissue interface impedance, delivered change, current, power, voltage, work, or the like. An additional neutral electrode patient ground patch (not shown) may be used to evaluate the desired bipolar electrical path impedance, as well as monitor and alert the operator upon detection of undesired and/or unsafe conditions. As used herein, the term "bipolar ablation" or "bipolar energy" may refer to the delivery of electric pulses between two electrodes (for example, between two electrodes 18 of the medical device 12), rather than between a single device electrode and a ground electrode (for example, as is the case in unipolar ablation). The generator 14 may be configured to deliver a sampling pulse prior to delivery of a full series or "pulse train" of pulsed electric field ablative therapy pulses. Such a preliminary sampling pulse may provide measurements of relative electrical impedance between electrodes and warning of inappropriate electrode configurations such as overlapping electrodes and/or electrodes that are positioned too closely together and that could result in, for example, a short circuit condition. The medical device 12 may be configured to deactivate certain electrodes 18 if they are overlapping and/or positioned too closely together. Additionally, such preliminary pulses may be used to evaluate such conditions as relative proximity of individual electrodes 18 to ensure an appropriate voltage is to be applied to the electrodes during subsequent energy delivery and the voltage that is delivered to the electrodes 18 may be adjusted. These preliminary pulses may also be applied to assess whether the electrodes are positioned properly relative to the target tissue allowing the electrodes 18 to be repositioned in relation to the target tissue. The preliminary pulses may be delivered with or without automated, immediate, subsequent delivery of one or more therapeutic pulse trains.

When the medical device 12 is initially positioned before initiation of a delivery of electroporation energy, one or more checks are typically performed to determine whether the expandable element 32 and/or electrodes 18 are optimally positioned to ablate an area of target tissue without causing unintended damage to non-target tissue and/or damage to the medical device 12 or generator 14. The check(s) may fail if the processing circuitry 60 determines that one or more alert conditions are present. In one non-limiting example, the medical device 12 may be navigated to a target treatment site to perform an electroporation procedure, such as electroporation of cardiac tissue, renal tissue, airway tissue, and/or organs or tissue within the cardiac space. Specifically, the expandable element 32 may be expanded or collapsed (in some embodiments, inflated or deflated) and may adjust to the shape of a particular tissue region. When the expandable element 32 is expanded or inflated, the electrodes may initially deliver a sampling pulse to measure, as a nonlimiting example, relative electrical impedance. Depending upon the impedance measurement, a warning may be provided (for example, an audible warning and/or a visual warning, such as a LED light or text or symbolic indication shown on one or more displays 56) to alert that certain electrodes 18 may not be properly positioned, which the processing circuitry 60 may identify as an alert condition. For example, certain electrodes 18 may be in contact with or proximate tissue that is not intended to be ablated or certain electrodes 18 may be too close to one another for safe delivery of energy. Any electrodes 18 that have an impedance measurement that triggers the warning may be deactivated so that energy will not be delivered to those particular electrodes 18. The medical device 12 may be repositioned and another sampling pulse may measure relative electrical impedance to determine if a warning is generated for any of the electrodes 18 when the medical device is 12 in the new position. In one embodiment, impedance measurements may be recorded by each electrode 18 at each of two frequencies (for example, 12 kHz and 100 kHz) and those impedance measurements for each electrode 18 may be compared to each other, to impedance measurement(s) from other electrode(s) 18, and/or to impedance measurements recorded by surface electrodes 62, and/or other system electrodes. If no warning is provided on the display 56, the electrodes 18 may be activated by the processing circuitry 60, thus being capable of transmitting energy from the generator 14. Additionally, or alternatively, when a warning is generated for one or more electrodes 18, the processing circuitry 60 may deactivate or prevent the delivery of electroporation energy to those electrodes 18 without requiring the medical device 12 to be repositioned.

As a further non-limiting example, the system 10 may perform a check to determine whether the expandable element 32 has been properly expanded or inflated and, therefore, to determine whether there is adequate spacing between adjacent electrodes 18 on each shaft 34 and adequate spacing between adjacent shafts 34 for the safe and/or effective delivery of energy. When the expandable element 32 is expanded or inflated prior to the delivery of energy, portions of the expandable element 32 may not expand as intended and/or may adhere together, which may cause adjacent electrodes 18 and/or shafts 34 to be located very close to each other. If energy were delivered in a bipolar fashion between electrodes without adequate spacing or that were in contact with each other, a spike in the delivered current may occur. Put another way, when there is uniform spacing between electrodes 18 and/or the shafts 34, the electric field between the electrodes 18 will have a uniform intensity. Additionally, delivering energy from electrodes 18 on an improperly expanded or inflated expandable element 32 (for example, an expandable element with impaired/compromised symmetry) may result in the formation of non-contiguous or non-transmural lesions. The processing circuitry 60 may identify such improper inflation as an alert condition. After the expandable element 32 is expanded (such as by inflation), a sampling pulse may be delivered to determine if the expandable element 32 has fully expanded/inflated. If there are portions of the expandable element 32 are adhering together, the impedance signal will be altered and the processing circuitry 60 may alert the user. The electrodes 18 that are associated with the altered impedance signal may be deactivated and/or the expandable element 32 may then be at least partially deflated/collapsed and then reinflated/re-expanded to full expansion. Alternatively, the medical device 12 may be removed from the patient and replaced with a new device. These checks enable the processing circuitry 60 to determine which electrode(s) 18 should be activated or deactivated, an inflation status of the expandable element 32 (for example, whether the expandable element has symmetrically expanded/inflated and/or whether the electrodes 18 are properly spaced from each other), whether to initiate the delivery of energy, and/or other parameters. In other words, if some of the electrodes 18 located on the shafts 34 are found to be in close proximity to each other after expansion of the expandable element 32, then the sampling pulse and impedance checks provide a warning and/or alert the user to re-expand the expandable element 32 to achieve the desired uniform electrode spacing.

Once the checks have been performed and the processing circuitry 60 determines the delivery of energy should be initiated and, optionally, to which electrode(s) 18, transmission of energy from the generator 14 to the electrode(s) 18 commences. The generator 14 may be configured and programmed to deliver pulsed, high-voltage electric fields appropriate for achieving reversible or irreversible electroporation. In some examples, the generator 14 may be configured to deliver irreversible electroporation energy that is sufficient to induce cell death for purposes of completely blocking an aberrant conductive pathway along or through cardiac tissue, destroying the ability of the cardiac tissue to propagate or conduct cardiac depolarization waveforms and associated electrical signals.

One or more electrodes 18 may be used to perform impedance measurements before, during, and/or after the delivery of electroporation energy. In one embodiment, the electrode(s) 18 that are activated and that transmit energy may be used to sense impedance in the area of tissue to which the energy is delivered. Additionally, or alternatively, the electrode(s) 18 that are deactivated and do not transmit energy may be used to sense impedance in nearby tissue and/or in surrounding fluid. The processing circuitry 60 may use the impedance measurements to determine if the tissue to which energy has been delivered (that is, the treated tissue) has been adequately ablated. The electrode(s) 18 may continue delivering energy, or may be reactivated to deliver energy, to area(s) of tissue from which impedance measurements have been recorded that indicate sufficient ablation has not occurred.

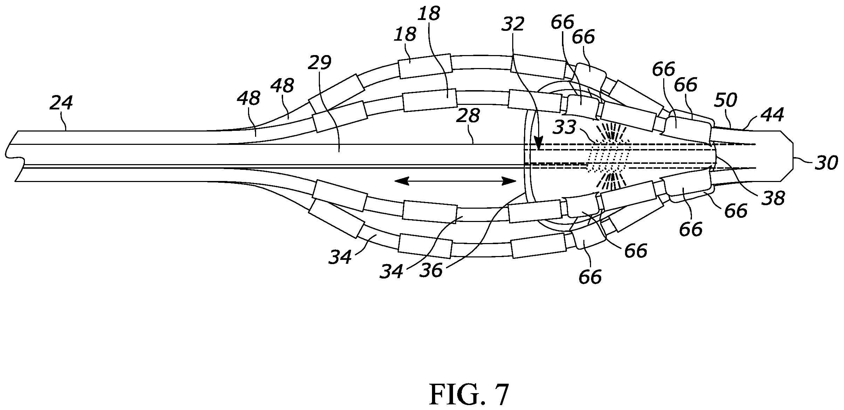

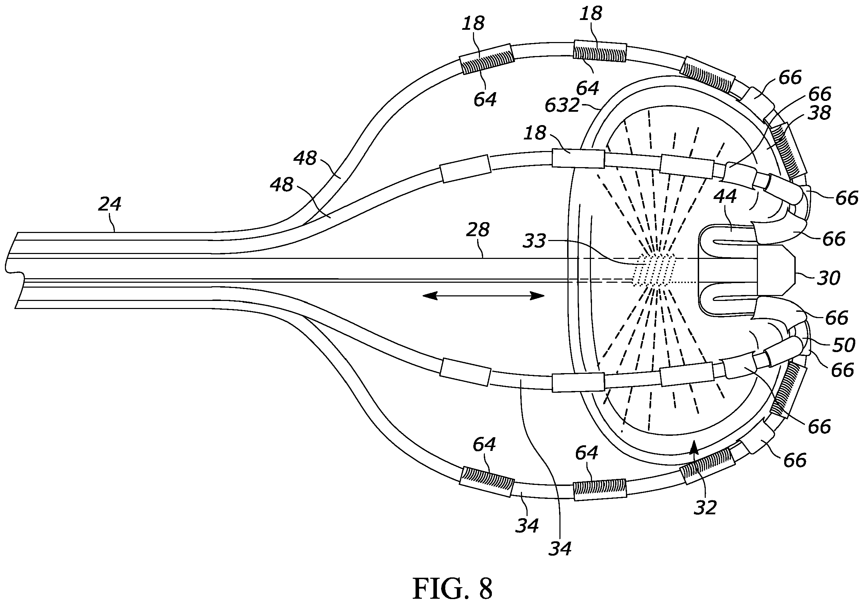

Now referring to FIG. 6, in some configurations, each electrode 18 disposed on the shafts 34 may be partially coated with a layer 64 of an electrically insulative material. In some examples, the insulative material is a high dielectric strength polymer or other suitable electrically insulative material such as, for example, Parylene, polyimide, metal oxide coating, diamond-like carbon, and the like. The entire inner surface of each electrode 18 may be insulated or only a portion of the inner surface may be insulated. Herein, the term "inner surface" refers to a portion of the electrode 18 that faces towards or is in direct contact with an outer wall (e.g., a skin or film) 632 of the expandable element 32. In embodiments in which an electrode 18 is not immediately adjacent to the expandable element 32, the inner surface of such electrode 18 includes portions of the electrode surface that are primarily oriented towards or are facing the (central) actuation element 28 (e.g., see FIG. 8). The purpose of the insulative layers 64 on the inner surface of some or all of the electrodes is to reduce electrical currents delivered into the blood pool that might be present in the gaps between the shafts 34 as opposed to delivering pulsed electrical current into the targeted tissue in contact with the outer surface of the electrodes 18. In some embodiments and/or geometric configurations, the layers 64 of all of the electrodes will be in contact with the outer wall 632 of the inflated expandable element 32. In some other embodiments and/or geometric configurations, where a smaller expandable element 32 is used (e.g., see FIGS. 7, 8, 9, and 10), some of the electrodes 18 will not be in contact with the outer wall 632 of the expandable element 32, which may cause the layers 64 of some of the electrodes 18 be in contact with the blood pool. However, the presence of the layers 64 will still beneficially limit the electrical currents directed through such blood pool without limiting the electrical currents directed through the targeted tissue.

Now referring to FIGS. 7-10, the expandable element 32 of the medical device 12 may have a smaller size configured to fill only the distal space near the distal ends 44 of the shafts 34. For example, as shown in FIG. 7, the plurality of flexible shafts 34 may together define a delimited volume located between the actuation element 28 and the flexed shafts 34. In some examples, the expandable element 32 is sufficiently small in size or diameter such that, when inflated or expanded, the expandable element 32 fills up or encompasses less than the entire delimited volume. In various examples, in the inflated state, the expandable element 32 fills up less than approximately 70%, 50%, or 30% of the delimited volume.

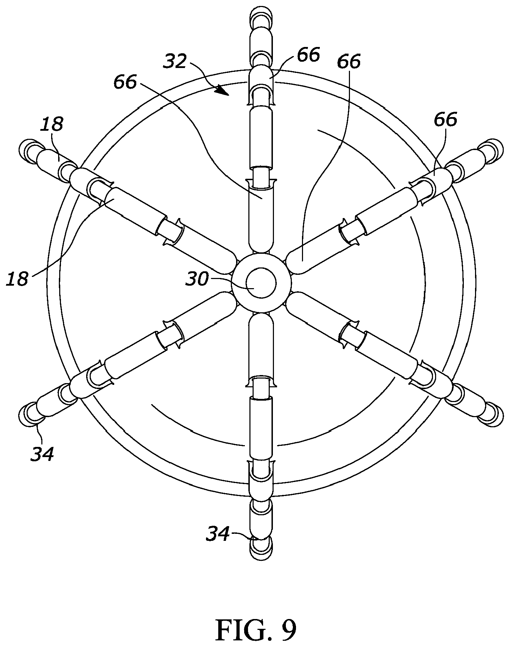

Additionally, as shown in FIGS. 7-9, the device 12 may include a plurality of shaft retention elements 66 coupled to the expandable element 32. Each shaft retention element 66 defines an open lumen sized and configured to circumscribe the outer surface of the corresponding shaft 34, thereby mechanically coupling the shaft 34 to the outer wall 632 of the expandable element 32, maintaining controlled distances or spacing between individual shafts 34, and maintaining the shafts 34 in a predetermined alignment or radial orientation when the expandable element 32 is inflated. The shaft retention elements 66 may be positioned proximate to the distal tip 30 and/or between adjacent electrodes 18 that are coupled to each shaft 34. In some examples, the expandable element 32 is only coupled to, or proximate to, the distal ends 44 of the shafts 34 and is proximate to the distal tip 30. As shown in FIG. 8, each retention element 66 may be flexible to contort and/or bend with the shafts 34 as the device 12 transitions from the above-described first (extended) configuration to the second (retracted) configuration. Further, as shown in FIG. 9, the plurality of retention elements 66 may include a first set of retention elements 66 having a first length and a second set of retention elements 66 having a second length greater than the first length of the first set of retention elements 66. In one embodiment, the larger second set of retention elements 66 may be positioned in closer proximity to the distal tip 30 such that each element 66 of the second set of retention elements is positioned between an electrode 18 and the distal tip 30 to provide greater structural support and integrity near the distal tip 30 when the device 12 transitions between the first (extended) configuration and the second (retracted) configuration. Although the retention elements 66 are only shown in the like drawings as being disposed around a smaller expandable element 32, it is to be understood that the plurality of retention elements 66 may also be coupled to a larger expandable element 32 shown in FIGS. 2-4.

In some configurations, the device 12 shown in FIGS. 7-9 may include a flexible joint in each of the shafts 34, located just proximal to the expandable element 32, such that upon insertion of the inflated expandable element 32 into a targeted cavity, such as a pulmonary vein ostium, combined with partial retraction of the actuation element 28, the expandable element 32 preferentially takes on a "pear-shape" which allows the distal portion of the expandable element 32 to compress to a smaller diameter and the proximal portion to expand to a larger diameter, thus applying the more-proximal electrodes 18 in contact with the antral wall of the left atrium surrounding the pulmonary vein.

In some configurations, the stiffness of the shafts 34 located in the distal portion of the expandable element 32 may further be actively modulated by way of super elastic stiffening wires within each shaft 34 which may be actively retracted or advanced within lumens disposed within each shaft 34, thus allowing the user or clinician to actively control the shaping of the distal smaller diameter portion allowing adjustment of the relative distal and proximal portions of the expandable element 32. This feature enables customized shaping of the expandable element 32 to fit different anatomies.

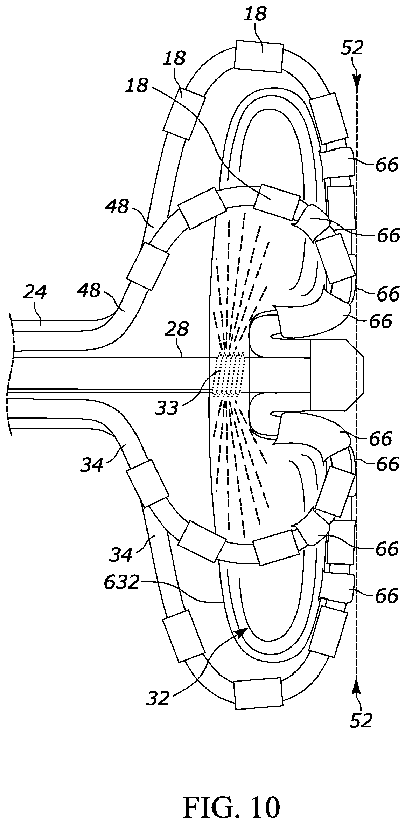

Now referring to FIG. 10, the actuation element 28 may be retracted even further than the retraction shown in FIG. 3 to provide a larger, or wider, distal face 52 with a larger tissue contact area when in contact with a flat, or substantially flat, area or wall of tissue, such as, for example, the posterior left atrium. This configuration may be referred to herein as a third retracted configuration. In this configuration, the increased retraction of the actuation element 28 causes the expandable element 32 to become more ovoid, with an increased outer diameter but shorter length. When in the third retracted configuration, because the outer diameter of the expandable element 32 increases to form the ovoid, the placement of the electrodes 18 in relation to the expandable element 32 may change. For example, when the ovoid is formed, the increased outer diameter of the expandable element 32 causes the shafts 34 to become increasingly stretched, which results in the electrodes 18 being shifted to locations more towards the distal tip 30. The device 12 can be readily transitioned between the first extended configuration, second retracted configuration, and the third retracted configuration in any order or sequence desired by the clinician by adjusting (e.g., pushing or pulling) the actuation element 28.

Further, it is to be understood that in any configuration, the expandable element 32 is not necessarily designed to occlude blood flow from the blood vessel in which it is positioned. Rather, in some embodiments, it is desirable to not occlude blood flow so that blood may pass around the expandable element 32 and past the shafts 34 or a portion of the shafts 34.

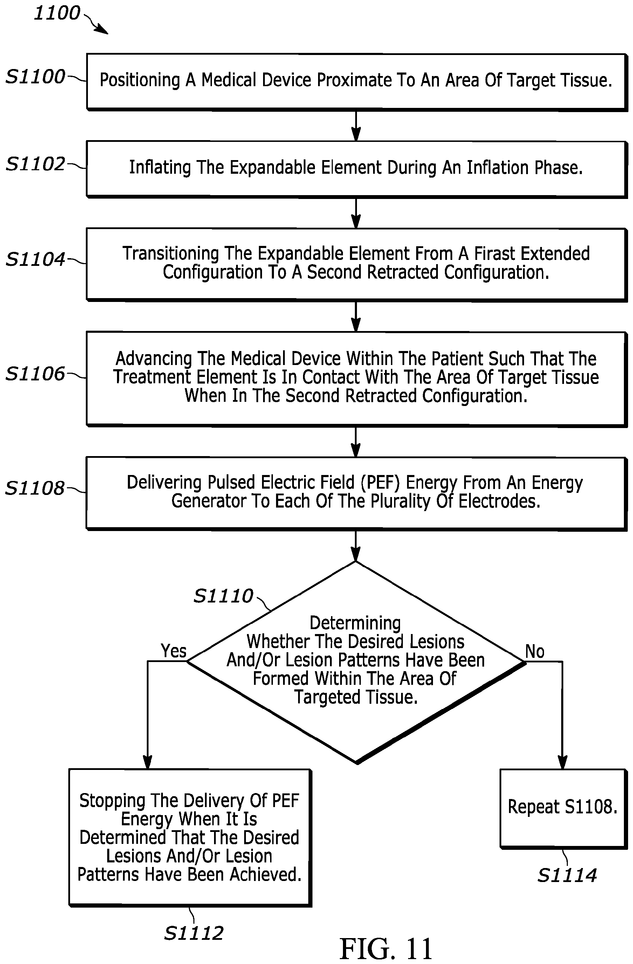

Now referring to FIG. 11, a flow chart describing an example method 1100 of using the treatment device 12 in accordance with the working principles of the system 10 described herein is shown. As mentioned above, prior to inflation of the expandable element 32, the medical device 12 is inserted within and navigated through the vasculature of a patient and positioned proximate to an area of target tissue (S1100). The area of target tissue may be an area of cardiac tissue, hepatic tissue, renal tissue, or bronchial tubes. Once it is determined that the device 12 is in a desired position, liquid, fluid, and/or gas is delivered to the device 12 from a supply source (see 101, FIG. 1A; 103, FIG. 1B; 172, FIG. 1C) to inflate or expand the expandable element 32 such that it transitions from a collapsed state to a first extended configuration (S1 102). Once the expandable element 32 is inflated to a desired or target internal pressure, the expandable element 32 may be transitioned to the second retracted configuration (shown in FIG. 3) by retracting the actuation element 28, thereby allowing the expandable element 32 to reach or be positioned at target locations not previously accessible when the expandable element 32 is in the first extended configuration (S1 104). The retraction of the actuation element 28 causes the distal tip 30 and distal ends 38 of each shaft 34 to be retracted towards the handle 46 such that the expandable element 32 forms a distal face 52 in which the distal tip 30 does not extend distally beyond the distal face 52. Once the device 12 is in the desired configuration (extended or retracted), the device 12 is further advanced within the patient such that the electrodes 18 are in contact with the area of target tissue (S1106). When it is detected that tissue contact has been achieved, pulsed electric field energy may then be delivered from the generator 14 to selected ones of the plurality of electrodes 18 during an ablation phase (S1108). Once the ablation procedure is complete, or it is determined that the desired lesions (or lesion patterns) have been formed within the area of target tissue (S1110), the delivery of pulsed electric field energy from the generator 14 is stopped automatically by the control unit 54 or manually by the clinician (S1112). However, if it is determined that the desired lesions and/or lesion patterns have not been formed within the area of target tissue, then the generator 14 is operated to once again initiate the ablation phase to re-treat the area of target tissue (S1114). Finally, the expandable element 32 is returned to its initial collapsed state and retracted through the patient's vasculature until it is removed from the patient's body.

FIGS. 12A-12B illustrate a braided shaft 34 used in the medical device 12 according to some examples. More specifically, FIG. 12A shows a longitudinal side view of the braided shaft 34. FIG. 12B shows a transverse cross-sectional view of the braided shaft 34. Example structural and electrical benefits of the use of the braided shafts 34 in the medical device 12 are already indicated above.

In the example shown, the braided shaft 34 includes three different types of strands braided together to form a braided jacket around a central elastic member 121. The first type of strands includes copper wires 122. The second type of strands includes constantan wires 123. The third type of strands includes PEN fibers 124. Both copper wires 122 and constantan wires 123 are individually insulated by having a thin insulting coat deposited onto the surface thereof. A total of four copper wires 122, four constantan wires 123, and eight PEN fibers 124 are braided to form the braided jacket around the central member 121. The eight PEN fibers 124 are arranged in one direction of the wrap, and the eight wires 122, 123 are arranged in the opposite direction of the wrap such that none of the strand-overlapping points in the braided jacket are conductor over conductor. This configuration of the braided jacket typically improves the electrical reliability of the braided shaft 34. In addition, the presence of copper wires 122 and constantan wires 123 in the braided shaft 34 enables the medical device 12 to have multiple *in situ* thermocouple junctions for temperature measurements in multiple points at the treatment site.

According to one example disclosed above, e.g., in the summary section and/or in reference to any one or any combination of some or all of FIGS. 1-12, provided is a medical treatment apparatus, comprising: an elongate body (e.g., 20, FIG. 3) including one or more lumens for mechanical, electrical, and fluid communication between a proximal portion (e.g., 22, FIG. 1A) and a distal portion (e.g., 24, FIG. 3), the distal portion including a plurality of flexible shafts (e.g., 34, FIG. 3) arranged around an actuation element (e.g., 28, FIG. 3) and coupled between the elongate body and a distal tip section (e.g., the section near the distal tip 30, FIG. 3) of the actuation element such that longitudinal movement of the actuation element with respect to the elongate body flexes the flexible shafts; an expandable element (e.g., 32, FIG. 3) attached to the distal tip section and positioned within a space delimited by the flexible shafts, the expandable element being connected via the one or more lumens to receive a fluid; and a plurality of electrodes (e.g., 18, FIG. 3) arranged along the flexible shafts and electrically connected via the one or more lumens to receive electrical energy for delivery to a target tissue.

In some examples of the above medical treatment apparatus, the medical treatment apparatus further comprises a plurality of retention elements (e.g., 66, FIG. 10), each of the retention elements being coupled between a respective one of the flexible shafts and the expandable element to anchor a corresponding portion of the expandable element to the respective one of the flexible shafts.

In some examples of any of the above medical treatment apparatus, the plurality of retention elements includes a first retention element located between the distal tip section and a first electrode of the plurality of electrodes on the respective one of the flexible shafts (e.g., as indicated in FIG. 10).

In some examples of any of the above medical treatment apparatus, the plurality of retention elements includes a second retention element located between the first electrode and a second electrode of the plurality of electrodes on the respective one of the flexible shafts (e.g., as indicated in FIG. 10).

In some examples of any of the above medical treatment apparatus, the expandable element is a balloon.

In some examples of any of the above medical treatment apparatus, a skin of the balloon is made of an electrically insulating material (e.g., a polymer).

In some examples of any of the above medical treatment apparatus, in an expanded state, the balloon takes up less than 70% by volume of the space delimited by the flexible shafts (e.g., as indicated in FIG. 10).

In some examples of any of the above medical treatment apparatus, in an expanded state, the balloon takes up less than 50% by volume of the space delimited by the flexible shafts (e.g., as indicated in FIG. 8).

In some examples of any of the above medical treatment apparatus, the medical treatment apparatus further comprises a fluid delivery conduit (e.g., 33, FIG. 3) connected via the one or more lumens to receive the fluid and configured to release the fluid from one or more openings, apertures, or ports thereof within the expandable element.

In some examples of any of the above medical treatment apparatus, the fluid delivery conduit has a segment thereof disposed circumferentially, spirally, or helically within the expandable element around the actuation element.

In some examples of any of the above medical treatment apparatus, the fluid delivery conduit is in fluid communication with a fluid supply source connected to the proximal portion.

In some examples of any of the above medical treatment apparatus, the fluid supply source comprises a pressurized bottle (e.g., 154, FIG. 1A; 164, FIG. 1B) filled with a fluid refrigerant or a gas.

In some examples of any of the above medical treatment apparatus, the fluid supply source comprises a syringe (e.g., 172, 174, FIG. 1C) filled with a physiological solution.

In some examples of any of the above medical treatment apparatus, the plurality of electrodes includes at least one electrode having a portion thereof facing the space delimited by the flexible shafts covered with a layer of an electrically insulating material (e.g., 64, FIGS. 6, 8).

In some examples of any of the above medical treatment apparatus, with the expandable element in an expanded state, the plurality of electrodes is configured to project electrical currents primarily away from the space delimited by the flexible shafts.

In some examples of any of the above medical treatment apparatus, the plurality of flexible shafts includes a braided shaft (e.g., 34, FIGS. 12A, 12B).

In some examples of any of the above medical treatment apparatus, the braided shaft comprises a plurality of braided strands including: a first type of strands comprising a first electrically conducting wire (e.g., 122, FIGS. 12A, 12B); and a second type of strands comprising an electrically insulating fiber (e.g., 124, FIGS. 12A, 12B).

In some examples of any of the above medical treatment apparatus, the plurality of braided strands further includes a third type of strands comprising a second electrically conducting wire (e.g., 122, FIGS. 12A, 12B), the first electrically conducting wire and the second electrically conducting wire comprising different respective electrically conducting materials.

In some examples of any of the above medical treatment apparatus, the braided shaft further comprises a central elastic member (e.g., 121, FIG. 12B); and wherein the braided strands form a braided jacket around the central elastic member.

In some examples of any of the above medical treatment apparatus, retraction of the of the actuation element moving the distal tip 30 toward the elongate body causes the plurality of flexible shafts to flex away from the actuation element thereby increasing in volume the space delimited by the flexible shafts (e.g., as evident from comparison of FIGS. 2 and 3).

Further discloses herein is a medical device. The medical device includes an elongate body, an expandable treatment element, a plurality of flexible shafts, and a plurality of electrodes. The elongate body has a proximal portion and a distal portion opposite the proximal portion. The expandable treatment element is coupled to the elongate body to receive a fluid and, in some examples, is anchored to the plurality of flexible shafts with a plurality of retention elements. In some examples, each flexible shaft of the plurality of flexible shafts has a braided configuration. Each electrode of the plurality of electrodes is attached and electrically coupled to a respective flexible shaft of the plurality of flexible shafts.

Aspects and embodiments of the invention may be defined by the following clauses.

Clause 1. A medical treatment apparatus, comprising:
an elongate body including one or more lumens for mechanical, electrical, and fluid communication between a proximal portion and a distal portion, the distal portion including a plurality of flexible shafts arranged around an actuation element and coupled between the elongate body and a distal tip section of the actuation element such that longitudinal movement of the actuation element with respect to the elongate body flexes the flexible shafts;
an expandable element attached to the distal tip section and positioned within a space delimited by the flexible shafts, the expandable element being connected via the one or more lumens to receive a fluid; and
a plurality of electrodes arranged along the flexible shafts and electrically connected via the one or more lumens to receive electrical energy for delivery to a target tissue.

Clause 2. The medical treatment apparatus of clause 1, further comprising a plurality of retention elements, each of the retention elements being coupled between a respective one of the flexible shafts and the expandable element to anchor a corresponding portion of the expandable element to the respective one of the flexible shafts.

Clause 3. The medical treatment apparatus of clause 2, wherein the plurality of retention elements includes a first retention element located between the distal tip section and a first electrode of the plurality of electrodes on the respective one of the flexible shafts.

Clause 4. The medical treatment apparatus of clause 3, wherein the plurality of retention elements includes a second retention element located between the first electrode and a second electrode of the plurality of electrodes on the respective one of the flexible shafts.

Clause 5. The medical treatment apparatus of clause 1, wherein the expandable element is a balloon.

Clause 6. The medical treatment apparatus of clause 5, wherein a skin of the balloon is made of an electrically insulating material.

Clause 7. The medical treatment apparatus of clause 5, wherein, in an expanded state, the balloon takes up less than 70% by volume of the space delimited by the flexible shafts.

Clause 8. The medical treatment apparatus of clause 5, wherein, in an expanded state, the balloon takes up less than 50% by volume of the space delimited by the flexible shafts.

Clause 9. The medical treatment apparatus of clause 1, further comprising a fluid delivery conduit connected via the one or more lumens to receive the fluid and configured to release the fluid from one or more openings, apertures, or ports thereof within the expandable element.

Clause 10. The medical treatment apparatus of clause 9, wherein the fluid delivery conduit has a segment thereof disposed circumferentially, spirally, or helically within the expandable element around the actuation element.

Clause 11. The medical treatment apparatus of clause 9, wherein the fluid delivery conduit is in fluid communication with a fluid supply source connected to the proximal portion.

Clause 12. The medical treatment apparatus of clause 11, wherein the fluid supply source comprises a pressurized bottle filled with a fluid refrigerant or a gas.

Clause 13. The medical treatment apparatus of clause 11, wherein the fluid supply source comprises a syringe filled with a physiological solution.

Clause 14. The medical treatment apparatus of clause 1, wherein the plurality of electrodes includes at least one electrode having a portion thereof facing the space delimited by the flexible shafts covered with a layer of an electrically insulating material.

Clause 15. The medical treatment apparatus of clause 1, wherein, with the expandable element in an expanded state, the plurality of electrodes is configured to project electrical currents primarily away from the space delimited by the flexible shafts.

Clause 16. The medical treatment apparatus of clause 1, wherein the plurality of flexible shafts includes a braided shaft.

Clause 17. The medical treatment apparatus of clause 16, wherein the braided shaft comprises a plurality of braided strands including:
a first type of strands comprising a first electrically conducting wire; and
a second type of strands comprising an electrically insulating fiber.

Clause 18. The medical treatment apparatus of clause 17, wherein the plurality of braided strands further includes a third type of strands comprising a second electrically conducting wire, the first electrically conducting wire and the second electrically conducting wire comprising different respective electrically conducting materials.

Clause 19. The medical treatment apparatus of clause 17,
wherein the braided shaft further comprises a central elastic member; and
wherein the braided strands form a braided jacket around the central elastic member.

Clause 20. The medical treatment apparatus of clause 1, wherein retraction of the of the actuation element causes the plurality of flexible shafts to flex away from the actuation element thereby increasing in volume the space delimited by the flexible shafts.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

It will be appreciated by persons skilled in the art that the present invention is not limited to what has been particularly shown and described herein above. In addition, unless mention was made above to the contrary, it should be noted that all of the accompanying drawings are not to scale. A variety of modifications and variations are possible in light of the above teachings without departing from the scope and spirit of the invention, which is limited only by the following claims.

## Claims

1. A medical treatment apparatus, comprising:
an elongate body including one or more lumens for mechanical, electrical, and fluid communication between a proximal portion and a distal portion, the distal portion including a plurality of flexible shafts arranged around an actuation element and coupled between the elongate body and a distal tip section of the actuation element such that longitudinal movement of the actuation element with respect to the elongate body flexes the flexible shafts;
an expandable element attached to the distal tip section and positioned within a space delimited by the flexible shafts, the expandable element being connected via the one or more lumens to receive a fluid; and
a plurality of electrodes arranged along the flexible shafts and electrically connected via the one or more lumens to receive electrical energy for delivery to a target tissue.

2. The medical treatment apparatus of claim 1, further comprising a plurality of retention elements, each of the retention elements being coupled between a respective one of the flexible shafts and the expandable element to anchor a corresponding portion of the expandable element to the respective one of the flexible shafts.

3. The medical treatment apparatus of claim 2, wherein the plurality of retention elements includes a first retention element located between the distal tip section and a first electrode of the plurality of electrodes on the respective one of the flexible shafts; and/or wherein the plurality of retention elements includes a second retention element located between the first electrode and a second electrode of the plurality of electrodes on the respective one of the flexible shafts.

4. The medical treatment apparatus of any preceding claim, wherein the expandable element is a balloon.

5. The medical treatment apparatus of claim 4, wherein a skin of the balloon is made of an electrically insulating material.

6. The medical treatment apparatus of claim 4, wherein, in an expanded state, the balloon takes up less than 70% by volume of the space delimited by the flexible shafts; or wherein, in an expanded state, the balloon takes up less than 50% by volume of the space delimited by the flexible shafts.

7. The medical treatment apparatus of any preceding claim, further comprising a fluid delivery conduit connected via the one or more lumens to receive the fluid and configured to release the fluid from one or more openings, apertures, or ports thereof within the expandable element.

8. The medical treatment apparatus of claim 7, wherein the fluid delivery conduit has a segment thereof disposed circumferentially, spirally, or helically within the expandable element around the actuation element.

9. The medical treatment apparatus of claim 7, wherein the fluid delivery conduit is in fluid communication with a fluid supply source connected to the proximal portion.

10. The medical treatment apparatus of claim 9, wherein the fluid supply source comprises a pressurized bottle filled with a fluid refrigerant or a gas; and/or wherein the fluid supply source comprises a syringe filled with a physiological solution.

11. The medical treatment apparatus of any preceding claim, wherein the plurality of electrodes includes at least one electrode having a portion thereof facing the space delimited by the flexible shafts covered with a layer of an electrically insulating material; and/or wherein, with the expandable element in an expanded state, the plurality of electrodes is configured to project electrical currents primarily away from the space delimited by the flexible shafts.

12. The medical treatment apparatus of any preceding claim, wherein the plurality of flexible shafts includes a braided shaft.

13. The medical treatment apparatus of claim 12, wherein the braided shaft comprises a plurality of braided strands including:
a first type of strands comprising a first electrically conducting wire; and
a second type of strands comprising an electrically insulating fiber.

14. The medical treatment apparatus of claim 13,
wherein the plurality of braided strands further includes a third type of strands comprising a second electrically conducting wire, the first electrically conducting wire and the second electrically conducting wire comprising different respective electrically conducting materials; and/or
wherein the braided shaft further comprises a central elastic member; and
wherein the braided strands form a braided jacket around the central elastic member.

15. The medical treatment apparatus of any preceding claim, wherein retraction of the actuation element causes the plurality of flexible shafts to flex away from the actuation element thereby increasing in volume the space delimited by the flexible shafts.
